(19) Europäisches Patentamt
European Patent Office
Office européen des brevets

(11) **EP 1 317 918 B1**

(12) # FASCICULE DE BREVET EUROPEEN

(45) Date de publication et mention de la délivrance du brevet:
**01.05.2019 Bulletin 2019/18**

(51) Int Cl.:
***A61Q 17/04** (2006.01)* ***A61K 8/44** (2006.01)*

(21) Numéro de dépôt: **02292840.2**

(22) Date de dépôt: **14.11.2002**

(54) **Compositions cosmétiques antisloaires base d'un mélange synergique de filtres et utilisations**

Kosmetische Sonnenschutzmittel auf Basis einer synergistischen Mischung von Filtern und deren Verwendungen

Cosmetic sunscreen compositions based on synergistic filter mixture and their use

(84) Etats contractants désignés:
**AT BE BG CH CY CZ DE DK EE ES FI FR GB GR IE IT LI LU MC NL PT SE SK TR**

(30) Priorité: **07.12.2001 FR 0115860**

(43) Date de publication de la demande:
**11.06.2003 Bulletin 2003/24**

(60) Demande divisionnaire:
**10154126.6 / 2 196 189**

(73) Titulaire: **L'Oréal**
**75008 Paris (FR)**

(72) Inventeur: **Candau, Didier**
**91570 Bievres (FR)**

(74) Mandataire: **Casalonga**
**Casalonga & Partners**
**Bayerstraße 71/73**
**80335 München (DE)**

(56) Documents cités:

**EP-A- 0 790 243** **EP-A- 0 893 119**
**EP-A- 1 046 391** **EP-A- 1 133 980**
**EP-A- 1 219 287** **EP-A1- 1 310 236**
**EP-A1- 1 310 239** **EP-A2- 1 290 999**
**EP-A2- 1 291 010** **EP-A2- 1 310 235**
**EP-A2- 1 310 237** **WO-A-95/22959**
**WO-A1-03/039502** **WO-A1-03/039507**
**DE-A1- 10 155 716** **DE-A1- 10 155 865**
**DE-A1- 10 155 900** **DE-A1- 10 157 484**
**DE-A1- 10 157 489** **DE-A1- 10 157 490**
**GB-A- 2 303 549**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 317 918 B1

Printed by Jouve, 75001 PARIS (FR)

## Description

L'invention concerne de nouvelles compositions cosmétiques ou dermatologiques à usage topique, en particulier pour la photoprotection de la peau et/ou des cheveux, caractérisées par le fait qu'elles comprennent, dans un support cosmétiquement acceptable, au moins :

(a) un filtre UV organique du type benzotriazole, insoluble sous forme micronisée de taille de particule allant de 10 nm à 5 μm, à titre de premier filtre et
(b) le 2-(4-diéthylamino-2-hydroxybenzoyl)-benzoate de n-hexyle, à titre de second filtre. L'association de ces deux filtres conduit à l'obtention d'un effet de synergie au niveau des facteurs de protection solaire UV-$A_{PPD}$ conférés.

L'invention concerne également leurs applications à la protection de la peau et des cheveux contre les effets du rayonnement ultraviolet.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

L'efficacité des compositions anti-solaires s'exprime généralement par le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Ce facteur concerne donc l'efficacité de la protection vis à vis de l'érythème dont le spectre d'action biologique est centré dans l'UVB et par conséquent, rend compte de la protection vis à vis de ce rayonnement UV-B.

Compte tenu des effets des UV-A sur la peau et du développement de nombreuses compositions contenant des associations de filtres capables d'absorber le rayonnement UV-B et/ou UV-A; il s'est développé des méthodes spécifiques d'évaluation de la protection contre le rayonnement UV-A.

Pour caractériser la protection vis à vis des UV-A, la méthode PPD (Persistent Pigment Darkening), qui mesure la couleur de la peau observée 2 à 4 heures après une exposition de la peau aux UV-A, est particulièrement recommandée et utilisée. Cette méthode est adoptée depuis 1996 par la Japanese Cosmetic Industry Association (JCIA) en tant que procédure officielle de test pour l'étiquetage UV-A des produits et est fréquemment utilisée par les laboratoires de tests en Europe et aux Etats-Unis; (Japan Cosmetic Industry Association Technical Bulletin. Measurement Standards for UVA protection efficacy. Issued November 21, 1995 and efective of January 1, 1996).

Le facteur de protection solaire $UVA_{PPD}$ (FP $UVA_{PPD}$) s'exprime mathématiquement par le rapport de la dose de rayonnement UV-A nécessaire pour atteindre le seuil de pigmentation avec le filtre UV ($MPPD_p$) avec la dose de rayonnement UV-A nécessaire pour atteindre le seuil de pigmentation sans filtre UV ($MPPD_{np}$).

$$\mathrm{FP\,UVA}_{\mathrm{PPD}} = \frac{\mathrm{MPPDp}}{\mathrm{MPPDnp}}$$

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction des facteurs de protection solaire recherchés.

On connaît dans les demandes de brevet EP-A-1046391 et DE10012408 des compositions antisolaires à base de dérivés de 2-hydroxybenzophénone aminosubstitués. Plus particulièrement, on connaît dans la demande de brevet WO 03/039507 des compositions antisolaires comprenant l'association d'un hydroxybenzophénol et d'au moins une triazine et/ou d'un dérivé de benzotriazole en présence d'additifs cosmétiques.

Or, à la suite d'importantes recherches menées dans le domaine de la photoprotection évoqué ci-dessus, la Demanderesse a découvert, de façon inattendue et surprenante, que la combinaison, dans des proportions comprises

dans des limites bien déterminées, de deux filtres solaires particuliers et déjà connus en soi dans l'état de l'art, permettait, du fait d'un effet de synergie remarquable, d'obtenir des compositions antisolaires présentant des facteurs de protection solaire UV-$A_{PPD}$ nettement améliorés, et en tous cas largement supérieurs à ceux qui peuvent être obtenus soit avec l'un ou l'autre des filtres utilisé seul.

Cette découverte est à la base de la présente invention.

Ainsi, conformément à l'un des objets de la présente invention, il est maintenant proposé de nouvelles compositions cosmétiques à usage topique, en particulier pour la photoprotection de la peau et/ou des cheveux, caractérisées par le fait qu'elles comprennent, dans un support cosmétiquement acceptable :

(a) au moins un filtre UV organique du type benzotriazole, insoluble sous forme micronisée de taille de particule allant de 10 nm à 5 μm, à titre de premier filtre et
(b) le 2-(4-diéthylamino-2-hydroxybenzoyl)-benzoate de n-hexyle, à titre de second filtre.

La présente invention a également pour objet l'utilisation d'une composition selon l'invention pour la fabrication de compositions cosmétiques ou dermatologiques destinées à la protection de la peau et/ou des cheveux contre le rayonnement ultraviolet, en particulier le rayonnement solaire.

Par filtre UV insoluble, au sens de la présente invention, on entend tout filtre UV organique ou minéral ayant une solubilité dans l'eau inférieure à 0,1 % en poids et une solubilité inférieure à 1 % en poids dans la plupart des solvants organiques comme l'huile de paraffine, les benzoates d'alcools gras et les triglycérides d'acides gras, par exemple le Miglyol® 812 commercialisé par la société DYNAMIT NOBEL. Cette solubilité, définie à 70 °C comme la quantité de produit en solution dans le solvant à l'équilibre avec un excès de solide en suspension, peut facilement être évaluée au laboratoire.

D'autres caractéristiques, aspects et avantages de la présente invention apparaîtront à la lecture de la description détaillée qui va suivre.

De façon générale, le filtre UV insoluble et le dérivé de 2-hydroxybenzophénone amino-substitué sont présents dans lesdites compositions dans une proportion produisant une activité synergique au niveau des facteurs de protection solaire UV-$A_{PPD}$ conférés.

Les filtres UV organiques insolubles selon l'invention ont une taille moyenne des particules qui varie de 10 à 5μm et plus préférentiellement de 10 nm à 2 μm et plus particulièrement de 20 nm à 2 μm.

Les filtres organiques insolubles selon l'invention peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée peuvent en particulier être obtenus par un procédé de broyage d'un filtre UV organique insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893119 faisant partie intégrante de la description. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure $C_nH_{2n+1}O(C_6H_{10}O_5)_xH$ dans laquelle n est un entier de 8 à 16 et x est le degré moyen de polymérisation de l'unité ($C_6H_{10}O_5$) et varie de 1,4 à 1,6. Ils peuvent être choisis parmi des esters en $C_1$-$C_{12}$ d'un composé de structure $C_nH_{2n+1}O(C_6H_{10}O_5)_xH$ et plus précisément un ester obtenu par réaction d'un acide carboxylique en $C_1$-$C_{12}$ tel que l'acide formique, acétique, propionique, butyrique, sulfosuccinique, citrique ou tartrique avec une ou plusieurs fonctions OH libres sur l'unité glucoside ($C_6H_{10}O_5$). Lesdits tensio-actifs sont utilisés en général à une concentration de allant de 1 à 50% en poids et plus préférentiellement de 5 à 40% en poids par rapport au filtre insoluble dans sa forme micronisée.

Les filtres UV organiques insolubles conformes à l'invention sont choisis notamment parmi les filtres UV organiques du type benzotriazole.

Parmi les filtres UV organiques insolubles du type benzotriazole conformes à l'invention, on peut citer ceux de formule suivante (3) tels que décrits dans la demande WO95/22959 (faisant partie intégrante du contenu de la description) :

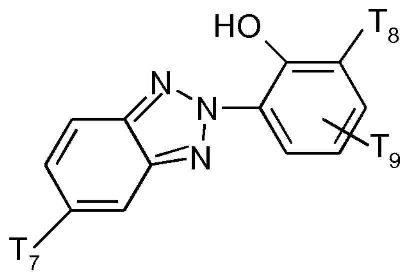

(3)

dans laquelle $T_7$ désigne un atome d'hydrogène ou un radical alkyle en $C_1$-$C_{18}$ ; $T_8$ et $T_9$, identiques ou différents, désignent un radical alkyle en $C_1$-$C_{18}$ éventuellement substitué par un phényle.

A titre d'exemple de composés de formule (3), on peut citer les produits commerciaux TINUVIN 328 , 320, 234 et 350 de la Société CIBA-GEIGY de structures suivantes :

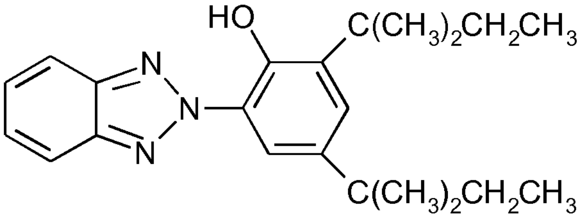

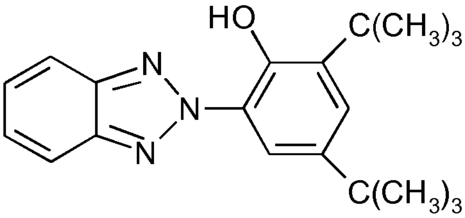

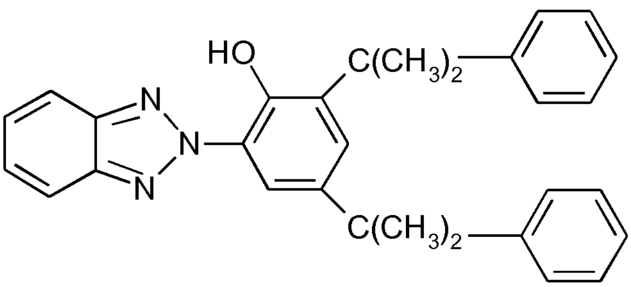

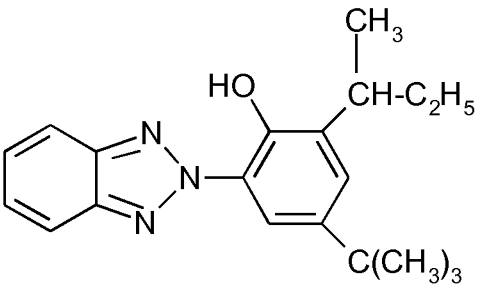

Parmi les filtres UV organiques insolubles du type benzotriazole conformes à l'invention, on peut citer les composés tels que décrits dans les brevets US 5 687 521, US 5 373 037, US 5 362 881 et en particulier le [2 ,4'-dihydroxy-3-(2H-benzotriazol-2-yl)-5-(1,1,3,3-tétraméthylbutyl)-2'-n-octoxy-5'-benzoyl] diphénylméthane vendu sous le

nom MIXXIM PB30 par la société FAIRMOUNT CHEMICAL de structure :

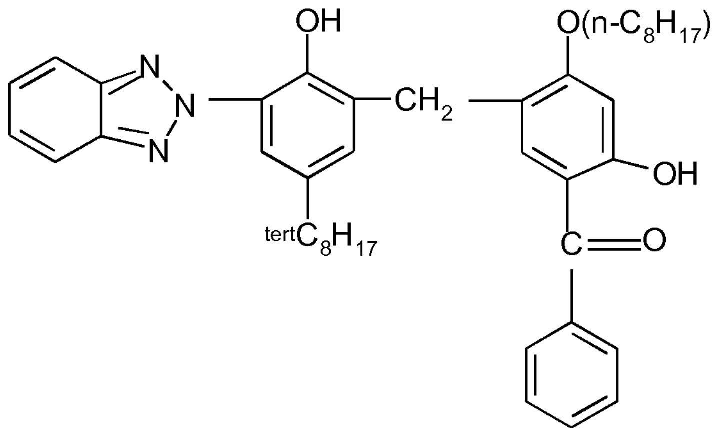

Parmi les filtres UV organiques insolubles du type benzotriazole conformes à l'invention, on peut citer les dérivés de méthylène bis-(hydroxyphényl benzotriazole) de structure suivante :

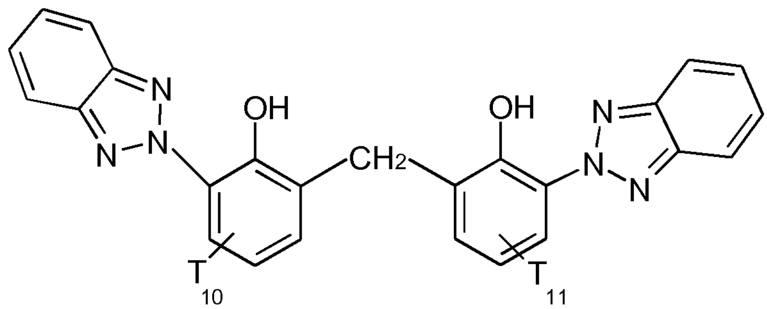

(4)

dans laquelle les radicaux $T_{10}$ et $T_{11}$, identiques ou différents, désignent un radical alkyle en $C_1$-$C_{18}$ pouvant être substitué par un ou plusieurs radicaux choisis parmi alkyle en $C_1$-$C_4$, cycloalkyle en $C_5$-$C_{12}$ ou un reste aryle. Ces composés sont connus en soi et décrits dans les demandes US 5237 071, US 5 166 355, GB-A-2 303 549, DE 197 26 184 et EP-A-893 119 (faisant partie intégrante de la description).

Dans la formule (4) définie ci-dessus : les groupes alkyle en $C_1$-$C_{18}$ peuvent être linéaires ou ramifiés et sont par exemple méthyle, éthyle, n-propyle, isopropyle, n-butyle, isobutyle, tert-butyle, tert-octyle, n-amyle, n-hexyle, n-heptyle, n-octyle, iso-octyle, n-nonyle, n-décyle, n-undécyle, n-dodécyle, tétradécyle, hexadécyle, ou octadécyle ; les groupes cycloalkyle en $C_5$-$C_{12}$ sont par exemple cyclopentyle, cyclohexyle, cyclooctyle ; les groupes aryle sont par exemple phényle, benzyle.

Parmi les composés de formule (4), on préfère plus particulièrement ceux de structures suivantes :

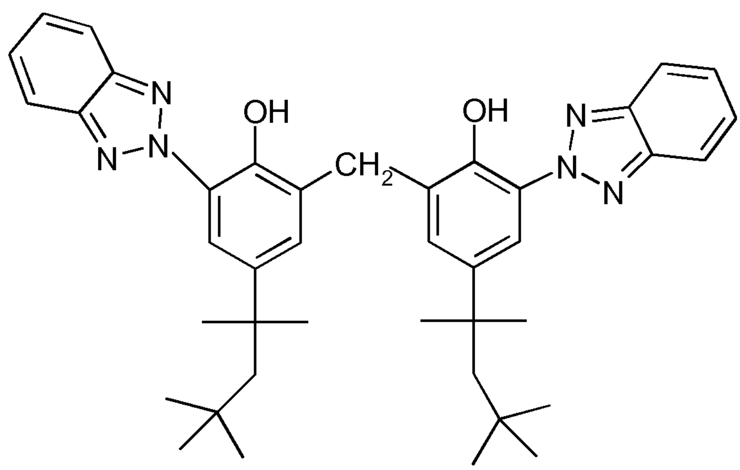

composé (a)

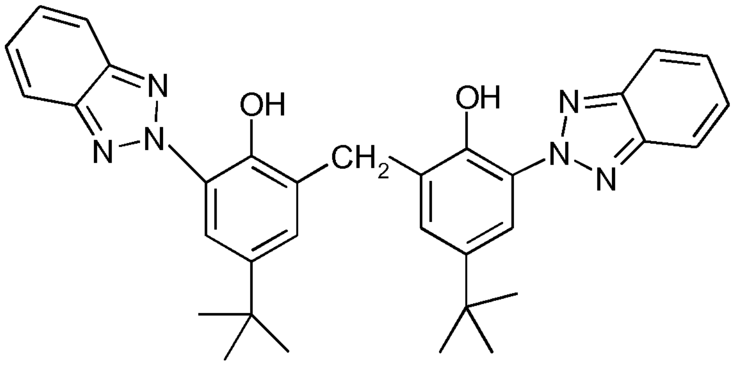

composé (b)

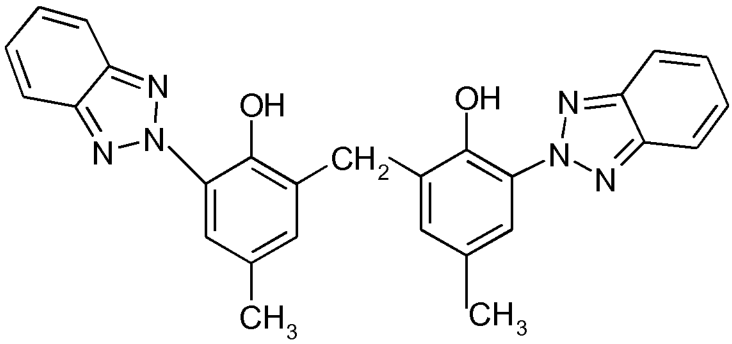

composé (c)

Le composé (a) de nomenclature 2,2'-méthylène-bis-[6-(2H-benzotriazol-2-yl)-4-(1,1,3,3-tetraméthylbutyl)phénol] vendu sous forme micronisée sous le nom TINOSORB M par la société CIBA SPECIALTY CHEMICALS.

Le composé (c) de nomenclature 2,2'-méthylène-bis-[6-(2H-benzotriazol-2-yl)-4-(methyl)phénol] est vendu sous forme solide sous le nom MIXXIM BB/200 par le société FAIRMOUNT CHEMICAL.

Le ou les filtres UV insolubles de l'invention sont présents à une concentration totale allant de préférence de 1 et 10 % en poids environ et plus particulièrement de 2 à 8% en poids par rapport au poids total de la composition.

Au sens de l'invention, les dérivés de 2-hydroxybenzophénone aminosubstitués répondent à la formule (I) suivante :

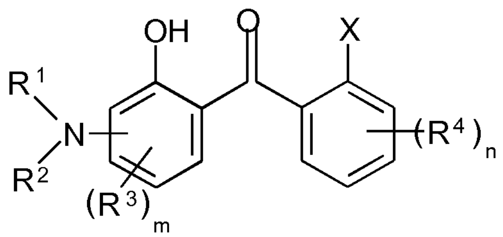

(I)

dans laquelle :

- $R^1$ et $R^2$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$ ;
- $R^1$ et $R^2$ peuvent également former avec l'atome d'azote avec lequel ils sont liés un cycle à 5 ou 6 chaînons ;
- $R^3$ et $R^4$, identiques ou différents, désignent un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un radical alcoxy en C1-C12, un radical ($C_1$-$C_{20}$)alcoxy-carbonyle, un radical alkylamino en $C_1$-$C_{12}$, un radical dialkylamino en $C_1$-$C_{12}$, un radical aryle ou un hétéroaryle éventuellement substitué, un substituant hydrosolubilisant choisi parmi un groupe carboxylate, un groupe sulfonate ou un reste ammonium ;
- X désigne un atome d'hydrogène, un groupe $COOR^5$ ou $CONR^6R^7$ ;
- $R^5$, $R^6$ et $R^7$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un groupe $-(YO)_o-Z$ ou un groupe aryle ;
- Y désigne $-(CH_2)_2-$, $-(CH_2)_3-$ $-(CH_2)_4-$, $-CH-(CH_3)-CH_2-$ ;

Z représente $-CH_2-CH_3$, $-CH_2CH_2CH_3$, $-CH_2-CH_2-CH_2-CH_3$, $-CH(CH_3)-CH_3$ ;
m est un entier variant de 0 à3 ;
n est un entier variant de 0 à 3 ;
o est un entier variant de 1 à 2.

Comme radicaux alkyle en $C_1$-$C_{20}$, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en $C_2$-$C_{10}$, on peut citer par exemple : vinyle, n-propènyle, isopropènyle, 1-butènyle, 2-butènyle, 1-pentènyle, 2-pentènyle, 2-méthyl-1-butènyle, 2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en $C_1$-$C_{12}$, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en $C_3$-$C_{10}$, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en $C_3$-$C_{10}$ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) chois par exemple parmi halogène comme chlore, fluor ou brome ; cyano ; nitro ; amino ; $C_1$-$C_4$-alkylamino ; $C_1$-$C_4$ dialkylamino ; $C_1$-$C_4$alkyle ; $C_1$-$C_4$-alcoxy ; hydroxy ; ils peuvent également comporter de 1 à 3 hétéroatomes comme souffre, oxygène ou azote dont les valences libres peuvent être occupées par un hydrogène ou un radical alkyle en $C_1$-$C_4$.

Les groupes aryles sont de préférence choisis parmi les cycles phényle ou naphtyle, lesquels pouvant comporter un ou plusieurs substituants (de préférence de 1 à 3) chois par exemple parmi halogène comme chlore, fluor ou brome ; cyano ; nitro ; amino ; $C_1$-$C_4$-alkylamino ; $C_1$-$C_4$ dialkylamino ; $C_1$-$C_4$alkyle ; $C_1$-$C_4$-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux $R^1$ et $R^2$ avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

**[0046]** Une famille de composés de formule (I) préférentiels comprend ceux choisis parmi ceux de formule (Ia) suivante :

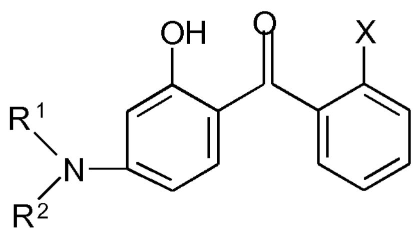

(Ia)

dans laquelle :

$R^1$ et $R^2$, identiques ou différents, désignent un atome d'hydrogène, un radical alkyle en $C_1$-$C_{12}$ ou forment avec l'atome d'azote avec lequel ils sont liés un cycle à 5 ou 6 chaînons ;
X désigne $COOR^5$ ou $CONR^6R^7$ ;
$R^5$ désigne un atome d'hydrogène, un radical alkyle en $C_1$-$C_{12}$, un radical cycloalkyle en $C_3$-$C_6$.
$R^6$ et $R^7$, identiques ou différents, désignent un atome d'hydrogène, un radical alkyle en $C_1$-$C_{12}$, un radical cycloalkyle en $C_5$-$C_6$.

Les composés de formule (Ia) plus particulièrement préférés sont ceux pour lesquels :

$R^1$ et $R^2$, identiques ou différents, désignent un radical alkyle en $C_1$-$C_4$ et plus particulèrement éthyle ;
$R^5$ désigne un radical alkyle en $C_3$-$C_8$,
$R^6$ et $R^7$, identiques ou différents, désignent un radical alkyle en $C_1$-$C_8$,

Une autre famille de composés de formule (I) préférentiels comprend ceux choisis parmi ceux de formule (Ib) suivante :

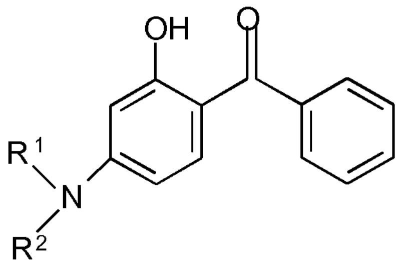

(Ib)

dans laquelle :
$R^1$ et $R^2$, identiques ou différents, désignent un radical alkyle en $C_1$-$C_{12}$ ou forment avec l'atome d'azote avec lequel ils sont liés un cycle à 5 ou 6 chaînons.

Parmi les composés de formule (Ib), on peut citer plus particulièrement :

- le (4-diéthylamino-2-hydroxyphényl)-phénylcétone.
- le (4-pyrrolidino-2-hydroxyphényl)-phénylcétone.

Une famille de composés de formule (I) plus particulièrement préférés comprend ceux choisis parmi ceux de formule (Ic) suivante :

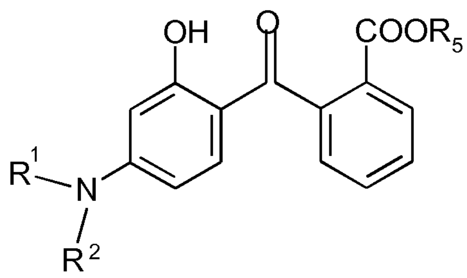

(Ic)

dans laquelle :

$R^1$ et $R^2$, identiques ou différents, désignent un atome d'hydrogène, un radical alkyle en $C_1$-$C_8$ ou forment avec l'atome d'azote avec lequel ils sont liés un cycle à 5 ou 6 chaînons ;
$R^5$ désigne un atome d'hydrogène, un radical alkyle en $C_1$-$C_{12}$, un radical cycloalkyle en $C_3$-$C_6$.

Parmi les composés de formule (Ic), on peut citer :

- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle

- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

Un composé de formule (I) tout particulièrement préféré est le 2-(4-diéthylamino-2-hydroxybenzoyl)-benzoate de n-hexyle.

Les composés de formule (I) tels que définis ci-dessus sont connus en eux-mêmes et leurs structures et leurs synthèses sont décrites dans les demandes de brevet EP-A-1046391 et DE100 12 408 (faisant partie intégrante du contenu de la description).

Les dérivés de 2-hydroxybenzophénone aminosubstitués conformes à l'invention sont présents de préférence dans la composition de l'invention dans des proportions allant de préférence de 0,1 à 20% en poids et plus préférentiellement de 0,1 à 15% en poids et plus particulièrement de 0,5 à 10% en poids par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles.

Les filtres UV organiques complémentaires sont notamment choisis parmi les anthranilates ; les dérivés cinnamiques ; les dérivés salicyliques, les dérivés du camphre autres que le composé (II) ; les dérivés de triazine tels que ceux décrits dans les demandes de brevet US 4367390, EP863145, EP517104, EP570838, EP796851, EP775698, EP878469 et EP933376 ; les dérivés de la benzophénone autres que ceux de formule (I) ; les dérivés de β,β'-diphénylacrylate ; les dérivés de benzotriazole ; les dérivés de benzalmalonate ; les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649; les 4,4-diarylbutadiènes tels que décrits dans les demandes EP0967200, DE19746654, DE19755649, EP-A-1008586.

Comme exemples de filtres organiques complémentaires actifs dans l'UV-A et/ou l'UV-B, on peut citer désignés ci-dessus sous leur nom INCI :

<u>Dérivés de l'acide para-aminobenzoique :</u>

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

<u>Dérivés salicyliques :</u>

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

<u>Dérivés du dibenzoylméthane :</u>

- Butyl Methoxydibenzoylmethane vendu notamment sous le nom commercial « PARSOL 1789 » par HOFFMANN LA ROCHE,
- Isopropyl Dibenzoylmethane,

Dérivés cinnamiques :

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,
- DEA Methoxycinnamate,
- - Diisopropyl Methylcinnamate,
- Glyceryl Ethylhexanoate Dimethoxycinnamate

Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial « UVINUL DS-49» par BASF,
- Benzophenone-12

Dérivé du benzylidène camphre :

- 3-Benzylidene camphor fabriqué sous le nom « MEXORYL SD» par CHIMEX,
- 4-Methylbenzylidene camphor vendu sous le nom « EUSOLEX 6300 » par MERCK,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- Polyacrylamidomethyl Benzylidene Camphor fabriqué sous le nom « MEXORYL SW » par CHIMEX,

Dérivés du phenyl benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial commercial « NEO HELIOPAN AP » par HAARMANN et REIMER,

Dérivés de la triazine :

- Anisotriazine vendu sous le nom commercial « TINOSORB S » par CIBA SPECIALTY CHEMICALS

- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés de benzalmalonate :

- Polyorganosiloxane à fonction benzalmalonate vendu sous la dénomination commerciale « PARSOL SLX » par HOFFMANN LA ROCHE et leurs mélanges.

Dérivés de 4,4-diarylbutadiene

1,1-dicarboxy(2,2'-diméthyl-propyl)-4,4-diphénylbutadiène.

Les filtres UV organiques solubles plus particulièrement préférés sont choisis parmi les composés suivants :

- Ethylhexyl Salicylate,
- Octocrylene,
- Ethylhexyl Methoxycinnamate
- Butyl Methoxydibenzoylmethane,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Drometrizole Trisiloxane,
- 1,1-dicarboxy (2,2'-diméthyl-propyl)-4,4-diphénylbutadiène

et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, en particulier l'effet de synergie, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Des exemples concrets, mais nullement limitatifs, illustrant l'invention, vont maintenant être donnés.

| Exemple 1 | |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en $C_{12}$-$C_{15}$ (WITCONOL TN -WITCO) | 10 g |
| 2-(4-diethylamino-2-hydroxybenzoyl)-benzoate de n-hexyle | 2g |
| Glycérine | 10g |
| Methylene bis-benzotriazolyl tetramethylbutylphenol (TINOSORB M - CIBA) | 10g |
| Conservateurs | qs |
| Eau déminéralisée qsp | 100 g |

| Exemple 2 (Référence) | |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en C12/C15 (WITCONOL TN -WITCO) | 15g |
| Triéthanolamine | 0.5g |
| 2-(4-diethylamino-2-hydroxybenzoyl)-benzoate de n-hexyle | 2.5g |
| Glycérine | 5g |
| Phosphate d'alcool hexadécylique,sel de potassium (AMPHISOL K - HOFFMAN LAROCHE) | 1g |
| Acide polyacrylique (SYNTHALEN K - 3V) | 0.3g |
| Hydroxypropyl méthyl cellulose (METHOCEL F4M -DOW CHEMICAL) | 0.1g |
| 2,2'-(1,4 phénylène)bis-benzoxazole | 4g |
| Triethanolamine | qs pH 7 |
| Conservateurs | qs |
| Eau démineralisée qsp | 100 g |

## Revendications

1. Composition cosmétique ou dermatologique à usage topique, en particulier pour la photoprotection de la peau et/ou des cheveux, **caractérisée par le fait qu'**elle comprend, dans un support cosmétiquement acceptable :

   (a) au moins un filtre UV organique du type benzotriazole, insoluble sous forme micronisée de taille de particule allant de 10 nm à 5 μm, à titre de premier filtre et
   (b) à titre de second filtre, le 2-(4-diethylamino-2-hydroxybenzoyl)-benzoate de n-hexyle

2. Composition selon la revendication 1, où les filtres UV organiques du type benzotriazole répondent à la formule (3) suivante :

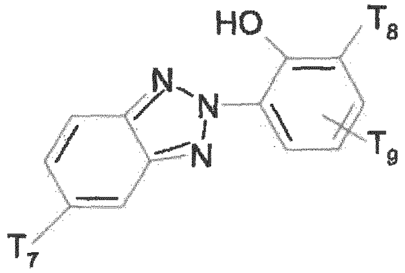

(3)

dans laquelle $T_7$ désigne un atome d'hydrogène ou un radical alkyle en $C_1$-$C_{18}$ ; $T_8$ et $T_9$, identiques ou différents, désignent un radical alkyle en $C_1$-$C_{18}$ éventuellement substitué par un phényle.

**3.** Composition selon la revendication 2, où le composé de formule (3) est choisi parmi les composés suivants :

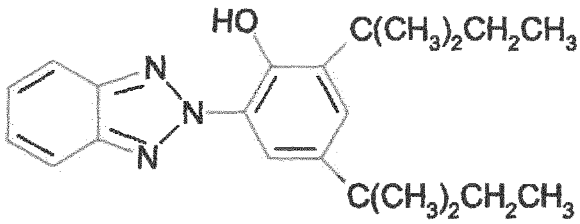

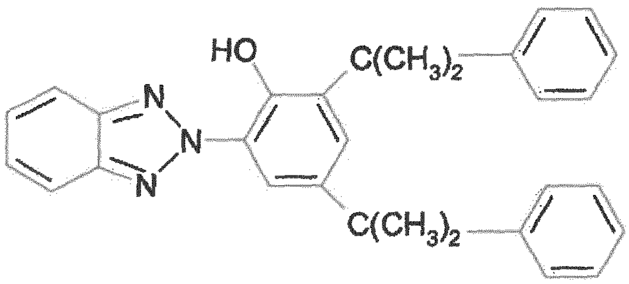

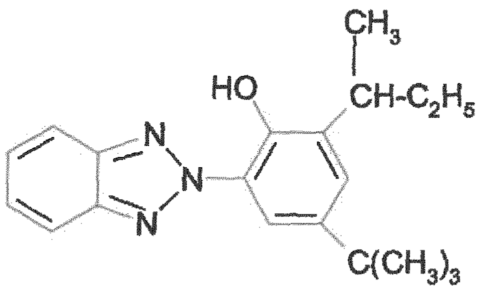

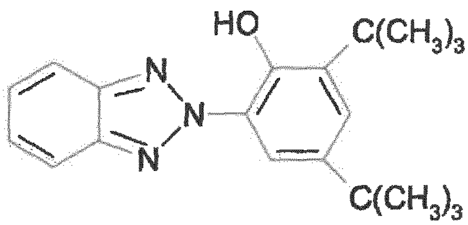

**4.** Composition selon la revendication 1, où le filtre UV insoluble est le [2 ,4'-dihydroxy-3-(2H-benzotriazol-2-yl)-5-(1,1,3,3-tétraméthylbutyl)-2'-n-octoxy-5'-benzoyl]diphénylméthane de structure :

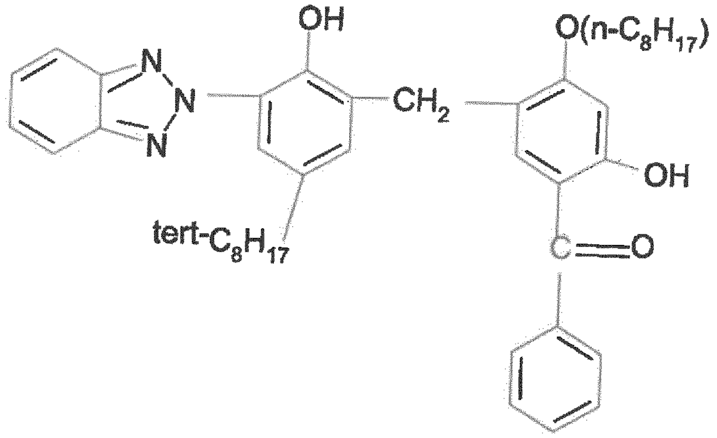

5. Composition selon la revendication 1, où les filtres UV organiques du type benzotriazole sont choisis parmi les dérivés de méthylène bis-(hydroxyphényl benzotriazole) de structure suivante :

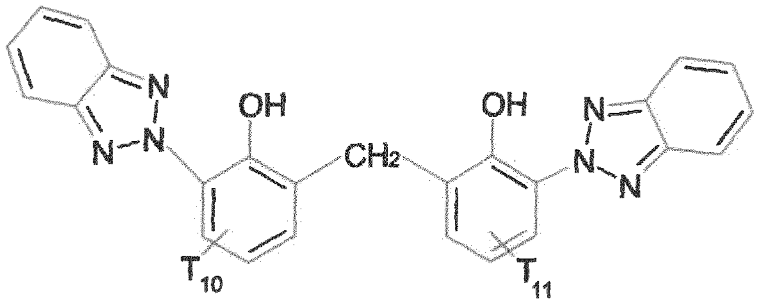

(4)

dans laquelle les radicaux $T_{10}$ et $T_{11}$ identiques ou différents, désignent un radical alkyle en $C_1$-$C_{18}$ pouvant être substitué par un ou plusieurs radicaux choisis parmi alkyle en $C_1$-$C_4$, cycloalkyle en $C_5$-$C_{12}$ ou un reste aryle.

6. Composition selon la revendication 5, où le composé de formule (4) est choisi dans le groupe constitué par les composés de structure suivante :

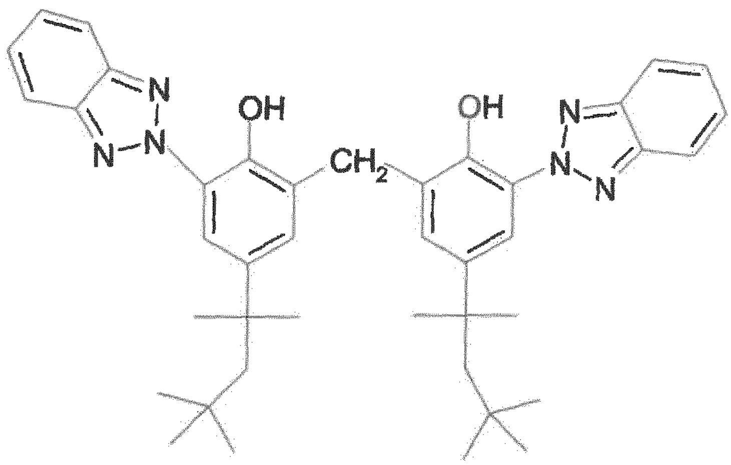

composé (a)

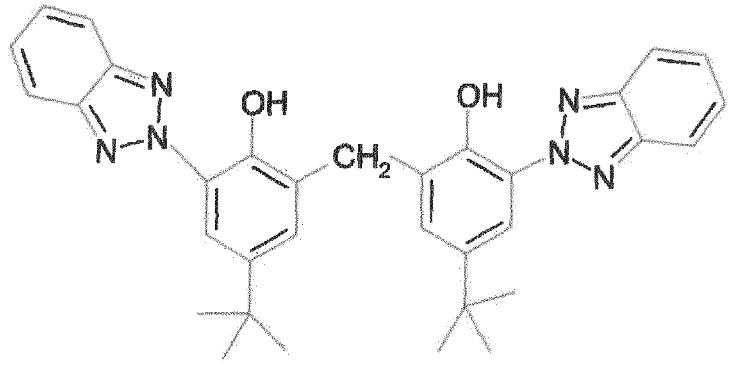

composé (b)

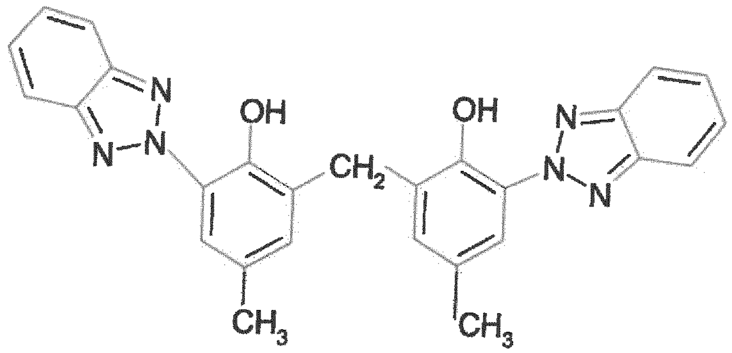

composé (c)

7. Composition selon l'une quelconque des revendications 1 à 6, où le 2-4-diéthylamino-2-hydroxybenzoyl)-benzoate de n-hexyle est présent dans la composition de l'invention dans des proportions allant de 0,1% à 20 % en poids, par rapport au poids total de la composition.

8. Composition selon la revendication 7 où le 2-(4-diéthylamino-2-hydroxybenzoyl)-benzoate de n-hexyle est présent dans la composition de l'invention dans des proportions allant de 0,1 % à 15 % en poids, par rapport au poids total de la composition,

9. Composition selon la revendication 8 où le 2-(4-diéthylamino-2-hydroxybenzoyl)-benzoate de n-hexyle est présents dans la composition de l'invention dans des proportions allant de 0,5 % à 20 % en poids, par rapport au poids total de la composition.

10. Composition selon l'une quelconque des revendications 1 à 9, **caractérisée par le fait qu'**elle comprend en outre au moins un agent de bronzage et/ou de brunissage artificiel de la peau.

11. Composition selon l'une quelconque des revendications 1 à 10, **caractérisée par le fait qu'**elle comprend en outre au moins un filtre UV organique soluble actif dans l'UV-A et/ou l'UV-B.

12. Composition selon la revendication 11, où les filtres UV organiques solubles sont choisis notamment parmi les anthranilates ; les dérivés cinnamiques ; les dérivés de dibenzoylméthane ; les dérivés salicyliques, les dérivés du camphre ; les dérivés de triazine ; les dérivés de la benzophénone autres que ceux de formule (I) ; les dérivés de β,β'-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les polymères filtres et silicones filtres ; les dimères dérivés d'α-alkylstyrène, les 4,4-diarylbutadiènes ; et leurs mélanges.

13. Composition selon la revendication 12, où les filtres UV organiques solubles sont choisis parmi :

- Ethylhexyl Salicylate,
- Octocrylene,
- Ethylhexyl Methoxycinnamate
- Butyl Methoxydibenzoylmethane,
- Phenylbenzimidazole Sulfonic Acid,

- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s-triazine.
- Drometrizole Trisiloxane,
- 1,1-dicarboxy(2,2'-diméthyl-propyl)-4,4-diphénylbutadiène,

et leurs mélanges.

**14.** Composition selon l'une quelconque des revendications 1 à 13, **caractérisée par le fait qu'**elle comprend en outre, des pigments ou des nanopigments d'oxydes métalliques, enrobés ou non.

**15.** Composition selon la revendication 14, **caractérisée par le fait que** lesdits pigments ou nanopigments sont choisis parmi les oxydes de titane, de zinc, de fer, de zirconium, de cérium et leurs mélanges, enrobés ou non.

**16.** Composition selon l'une quelconque des revendications 1 à 15, **caractérisée par le fait qu'**elle comprend en outre au moins un adjuvant cosmétique choisi parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

**17.** Composition selon l'une quelconque des revendications 1 à 16, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

**18.** Composition selon l'une quelconque des revendications 1 à 17, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

**19.** Composition selon l'une quelconque des revendications 1 à 17, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

**20.** Utilisation d'une composition telle que définie dans les revendications 1 à 17 pour la fabrication de compositions cosmétiques ou dermatologiques destinées à la protection de la peau et/ou des cheveux contre le rayonnement ultraviolet, en particulier le rayonnement solaire.

## Patentansprüche

**1.** Kosmetische oder dermatologische Zusammensetzung zur topischen Anwendung, insbesondere für den Lichtschutz der Haut und/oder der Haare,
**dadurch gekennzeichnet, dass** diese, in einem kosmetisch annehmbaren Träger, umfasst:

(a) mindestens einen organischen UV-Filter des Typs Benzotriazol, unlöslich in mikronisierter Form mit einer Partikelgröße von 10 nm bis 5 $\mu$m, als ersten Filter und
(b) als zweiten Filter, n-Hexyl-2-(4-diethylamino-2-hydroxybenzoyl)-benzoat.

**2.** Zusammensetzung nach Anspruch 1,

wobei die organischen UV-Filter des Typs Benzotriazol die folgende Formel (3) aufweisen:

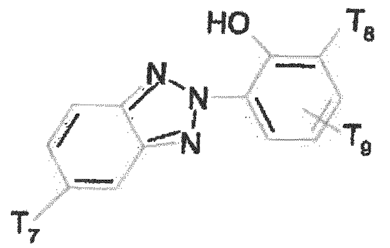

(3)

wobei $T_7$ ein Wasserstoffatom oder einen $C_1$-$C_{18}$-Alkylrest darstellt; $T_8$ und $T_9$, identisch oder verschieden, einen $C_1$-$C_{18}$-Alkylrest darstellen, gegebenenfalls durch substituiert durch ein Phenyl.

**3.** Zusammensetzung nach Anspruch 2,
wobei die Zusammensetzung mit der Formel (3) aus den folgenden Verbindungen ausgewählt ist:

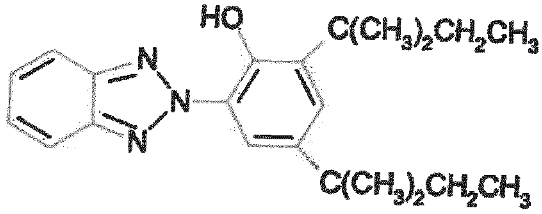

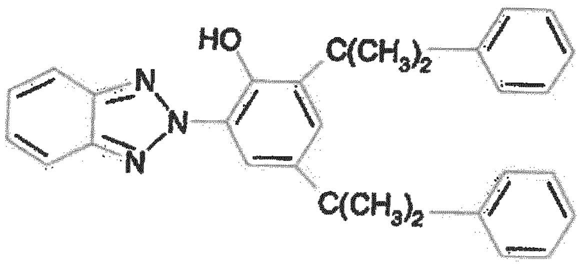

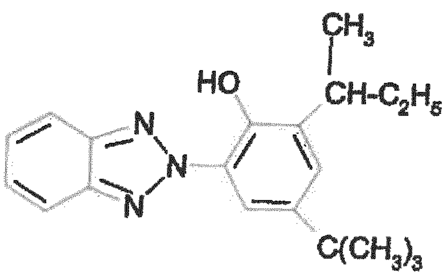

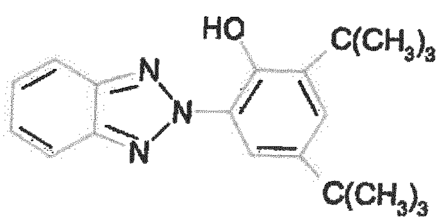

**4.** Zusammensetzung nach Anspruch 1,
wobei der unlösliche UV-Filter [2,4'-Dihydroxy-3-(2H-benzotriazol-2-yl)-5-(1,1,3,3-tetramethylbutyl)-2'-n-octoxy-5'-benzoyl]-diphenylmethan ist mit der Struktur:

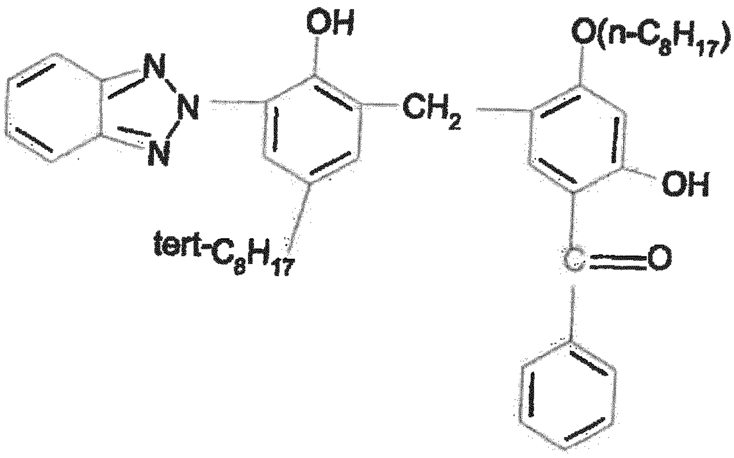

5. Zusammensetzung nach Anspruch 1, wobei die organischen UV-Filter des Typs Benzotriazol aus den Derivaten von Methylen-bis-(hydroxyphenylbenzotriazol) mit der folgenden Struktur ausgewählt sind:

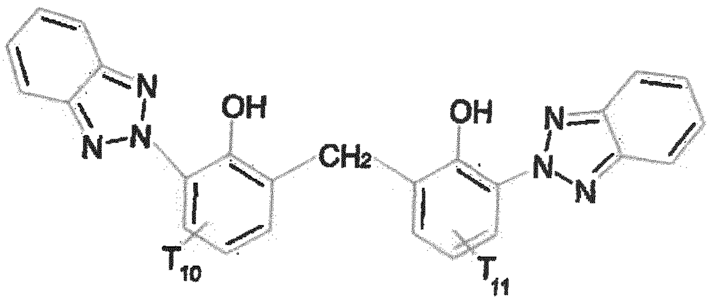

(4)

wobei die Reste $T_{10}$ und $T_{11}$, identisch oder verschieden, einen $C_1$-$C_{18}$-Alkylrest darstellen, der durch einen oder mehrere Reste substituiert sein kann, ausgewählt aus $C_1$-$C_4$-Alkyl, $C_5$-$C_{12}$-Cycloalkyl oder einem Arylrest.

6. Zusammensetzung nach Anspruch 5, wobei die Verbindung mit der Formel (4) ausgewählt ist aus der Gruppe bestehend aus den Verbindungen mit der folgenden Struktur:

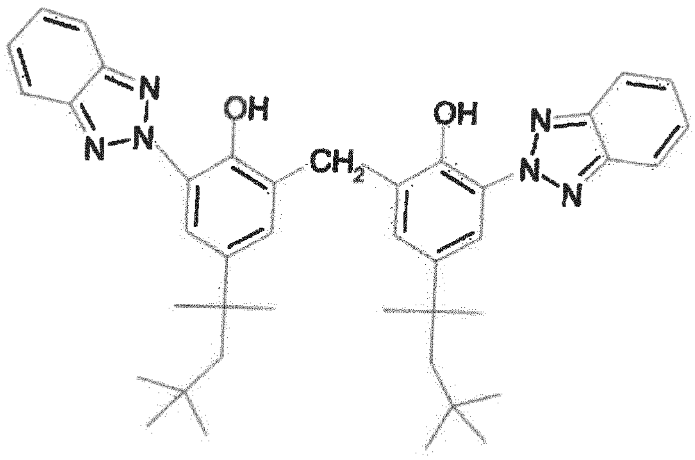

Verbindung (a)

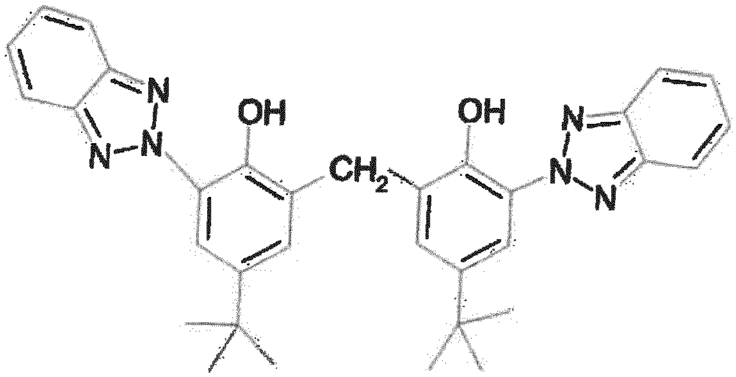

Verbindung (b)

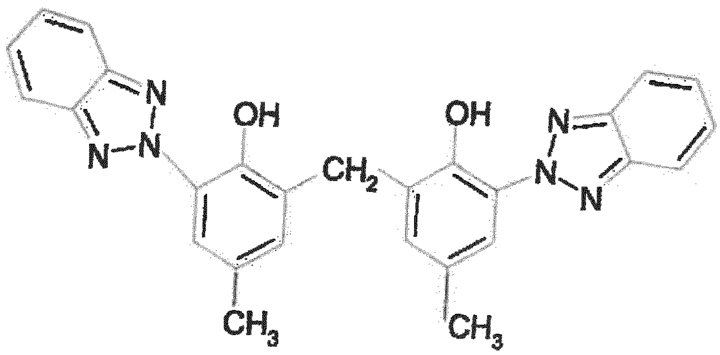

Verbindung (c)

7. Zusammensetzung nach einem der Ansprüche 1 bis 6, wobei das n-Hexyl-2-(4-diethylamino-2-hydroxybenzoyl)-benzoat in der Zusammensetzung der Erfindung in Anteilen von 0,1 Gew.-% bis 20 Gew.-%, bezogen auf das Gesamtgewicht der Zusammensetzung, vorliegt.

8. Zusammensetzung nach Anspruch 7, wobei das n-Hexyl-2-(4-diethylamino-2-hydroxybenzoyl)-benzoat in der Zusammensetzung der Erfindung in Anteilen von 0,1 Gew.-% bis 15 Gew.-%, bezogen auf das Gesamtgewicht der Zusammensetzung, vorliegt.

9. Zusammensetzung nach Anspruch 8, wobei das n-Hexyl-2-(4-diethylamino-2-hydroxybenzoyl)-benzoat in der Zusammensetzung der Erfindung in Anteilen von 0,5 Gew.-% bis 20 Gew.-%, bezogen auf das Gesamtgewicht der Zusammensetzung, vorliegt.

10. Zusammensetzung nach einem der Ansprüche 1 bis 9, **dadurch gekennzeichnet, dass** diese außerdem mindestens einen Bräuner und/oder ein künstliches Bräunungsmittel der Haut umfasst.

11. Zusammensetzung nach einem der Ansprüche 1 bis 10, **dadurch gekennzeichnet, dass** diese außerdem mindestens einen löslichen organischen UV-Filter aktiv in UV-A und/oder UV-B umfasst.

12. Zusammensetzung nach Anspruch 11, wobei die löslichen organischen UV-Filter insbesondere ausgewählt sind aus Anthranilaten; Zimtsäure-Derivaten; Dibenzoylmethan-Derivaten; Salicylsäure-Derivaten, Campher-Derivaten; Triazin-Derivaten; Benzophenon-Derivaten, die von jenen mit der Formel (I) verschieden sind; β,β'-Diphenylacrylat-Derivaten; Benzotriazol-Derivaten;

Benzalmalonat-Derivaten; Benzimidazol-Derivaten; Imidazolinen; bis-Benzoazolyl-Derivaten; p-Aminobenzoesäure-Derivaten (PABA); Polymerfiltern und Silikonfiltern; von α-Alkylstyrol stammenden Dimeren, 4,4-Diarylbutadienen; und ihren Mischungen.

**13.** Zusammensetzung nach Anspruch 12,
wobei die löslichen organischen UV-Filter ausgewählt sind aus:

- Ethylhexylsalicylat,
- Octocrylen,
- Ethylhexylmethoxycinnamat,
- Butylmethoxydibenzoylmethan,
- Phenylbenzimidazolsulfonsäure,
- Benzophenon-3,
- Benzophenon-4,
- Benzophenon-5,
- 4-Methylbenzylidencampher,
- Dinatriumphenyldibenzimidazoltetrasulfonat,
- Anisotriazin,
- Ethylhexyltriazon,
- Diethylhexylbutamidotriazon,
- 2,4,6-tris-(4'-Diisobtuylaminobenzalmalonat)-s-triazin,
- Drometrizoltrisiloxan,
- 1,1-Dicarboxy-(2,2'-dimethylpropyl)-4,4-diphenylbutadien,

und ihren Mischungen.

**14.** Zusammensetzung nach einem der Ansprüche 1 bis 13,
**dadurch gekennzeichnet, dass** diese außerdem Pigmente oder Nanopigmente von Metalloxiden, umhüllt oder nicht, umfasst.

**15.** Zusammensetzung nach Anspruch 14,
**dadurch gekennzeichnet, dass** die Pigmente oder Nanopigmente ausgewählt sind aus Titan-, Zink-, Eisen-, Zirconium-, Ceriumoxiden und ihren Mischungen, umhüllt oder nicht.

**16.** Zusammensetzung nach einem der Ansprüche 1 bis 15,
**dadurch gekennzeichnet, dass** diese außerdem mindestens ein kosmetisches Adjuvans umfasst, ausgewählt aus Fettkörpern, organischen Lösungsmitteln, ionischen oder nicht-ionischen Verdickungsmitteln, Weichmachern, Antioxidantien, Mitteln gegen freie Radikale, Trübungsmitteln, Stabilisatoren, Emollientien, Silikonen, α-Hydroxysäuren, Anti-Schaummitteln, Hydratisierungsmitteln, Vitaminen, Insektenabwehrmitteln, Parfums, Konservierungsmitteln, grenzflächenaktiven Stoffen, Entzündungshemmern, Substanz P-Antagonisten, Füllstoffen, Polymeren, Treibmitteln, Alkalinisierungs- oder Ansäuerungsmitteln, Färbemitteln.

**17.** Zusammensetzung nach einem der Ansprüche 1 bis 16,
**dadurch gekennzeichnet, dass** es sich um eine Zusammensetzung für den Schutz der menschlichen Epidermis oder um eine Sonnenschutzzusammensetzung handelt, und dadurch, dass diese in der Form einer nicht-ionischen vesikulären Dispersion, einer Emulsion, insbesondere einer Emulsion des Typs Ölin-Wasser, einer Creme, einer Milch, eines Gels, einer Gelcreme, einer Suspension, einer Dispersion, eines Pulvers, eines festen Stifts, eines Schaums oder eines Sprays dargereicht wird.

**18.** Zusammensetzung nach einem der Ansprüche 1 bis 17,
**dadurch gekennzeichnet, dass** es sich um eine Zusammensetzung zum Schminken der Wimpern, der Augenbrauen oder der Haut handelt, und dadurch, dass diese in fester oder Pastenform, wasserfrei oder wässrig, einer Emulsion, einer Suspension oder einer Dispersion dargereicht wird.

**19.** Zusammensetzung nach einem der Ansprüche 1 bis 17,
**dadurch gekennzeichnet, dass** es sich um eine Zusammensetzung handelt, die für den Schutz der Haare gegen Ultraviolettstrahlen bestimmt ist, und dadurch, dass diese in der Form eines Shampoos, einer Lotion, eines Gels, einer Emulsion einer nicht-ionischen vesikulären Dispersion dargereicht wird.

**20.** Verwendung einer Zusammensetzung, wie in den Ansprüchen 1 bis 17 definiert, zur Herstellung kosmetischer oder dermatologischer Zusammensetzungen, die für den Schutz der Haut und/oder der Haare gegen Ultraviolettstrahlung, insbesondere Sonnenstrahlung, bestimmt sind.

## Claims

**1.** Cosmetic or dermatological composition for topical use, in particular for photoprotecting the skin and/or the hair, **characterized in that** it comprises, in a cosmetically acceptable support:

(a) at least one organic UV-screening agent of the benzotriazole type, which is insoluble in micronized form with a particle size ranging from 10 nm to 5 $\mu$m, as first screening agent, and
(b) as second screening agent, n-hexyl 2-(4-diethylamino-2-hydroxybenzoyl)benzoate.

**2.** Composition according to Claim 1, in which the organic UV-screening agents of the benzotriazole type correspond to formula (3) below:

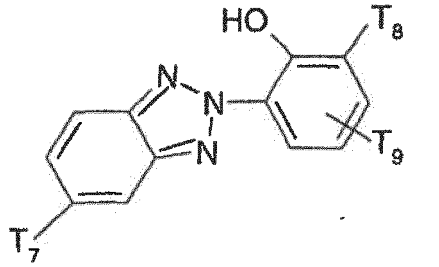

(3)

in which $T_7$ denotes a hydrogen atom or a $C_1$-$C_{18}$ alkyl radical; $T_8$ and $T_9$, which may be identical or different, denote a $C_1$-$C_{18}$ alkyl radical optionally substituted with a phenyl.

**3.** Composition according to Claim 2, in which the compound of formula (3) is chosen from the following compounds:

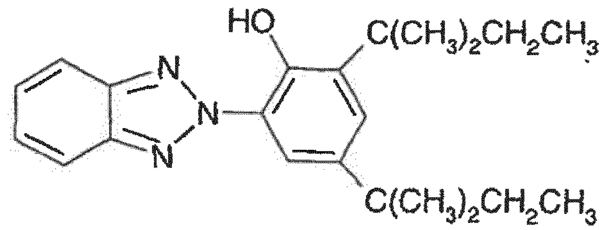

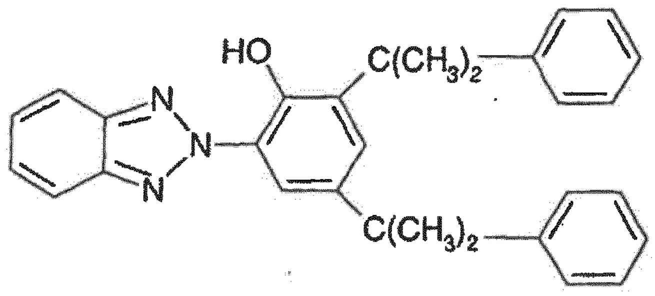

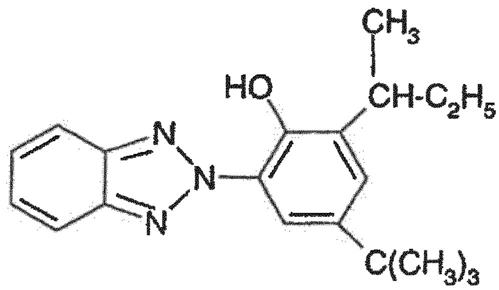

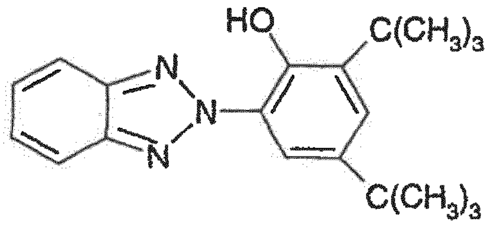

4. Composition according to Claim 1, in which the insoluble UV-screening agent is [2,4'-dihydroxy-3-(2H-benzotriazol-2-yl)-5-(1,1,3,3-tetramethylbutyl)-2'-n-octoxy-5'-benzoyl]diphenylmethane of structure:

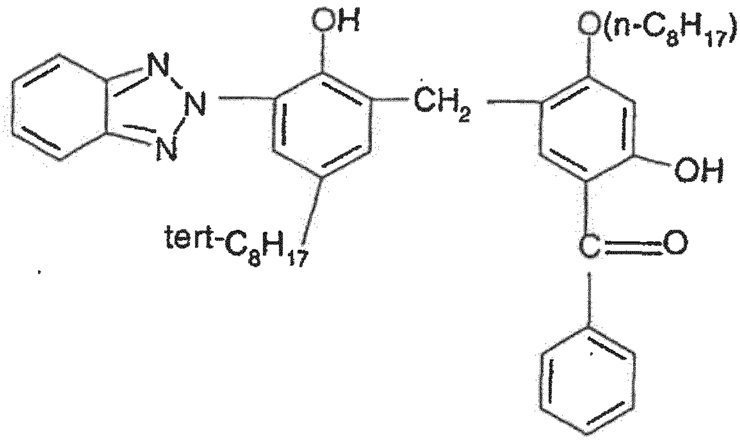

5. Composition according to Claim 1, in which the organic UV-screening agents of the benzotriazole type are chosen from the methylenebis(hydroxyphenylbenzotriazole) derivatives having the following structure:

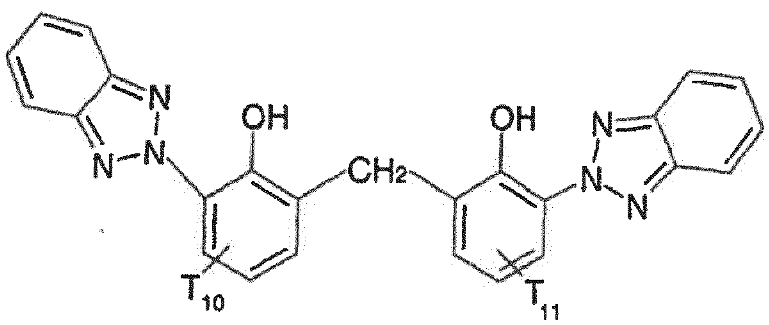

(4)

in which the radicals $T_{10}$ and $T_{11}$, which may be identical or different, denote a $C_1$-$C_{18}$ alkyl radical possibly substituted with one or more radicals chosen from $C_1$-$C_4$ alkyl, $C_5$-$C_{12}$ cycloalkyl and an aryl residue.

6. Composition according to Claim 5, in which the compound of formula (4) is chosen from the group consisting of the

compounds having the following structures:

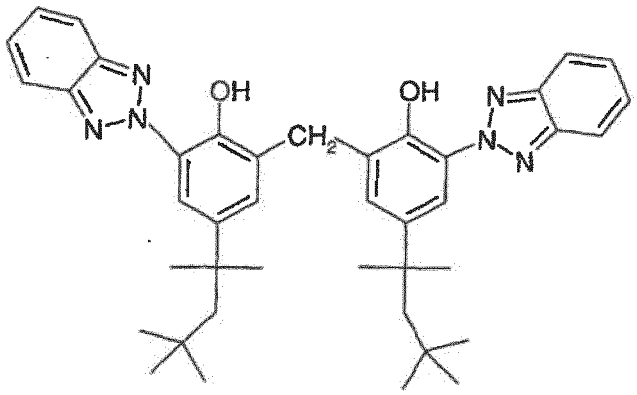

compound (a)

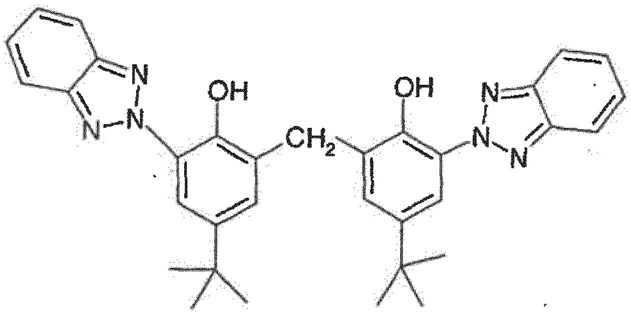

compound (b)

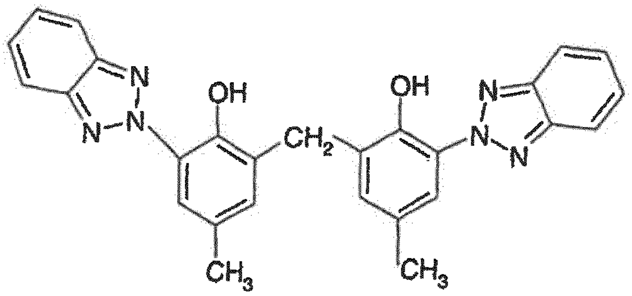

compound (c)

7. Composition according to any one of Claims 1 to 6, in which the n-hexyl 2-(4-diethylamino-2-hydroxybenzoyl)benzoate is present in the composition of the invention in proportions ranging from 0.1% to 20% by weight relative to the total weight of the composition.

8. Composition according to Claim 7, in which the n-hexyl 2-(4-diethylamino-2-hydroxybenzoyl)benzoate is present in the composition of the invention in proportions ranging from 0.1% to 15% by weight relative to the total weight of the composition.

9. Composition according to Claim 8, in which the n-hexyl 2-(4-diethylamino-2-hydroxybenzoyl)benzoate is present in the composition of the invention in proportions ranging from 0.5% to 20% by weight relative to the total weight of the composition.

10. Composition according to any one of Claims 1 to 9, **characterized in that** it also comprises at least one agent for artificially tanning and/or browning the skin.

11. Composition according to any one of Claims 1 to 10, **characterized in that** it also comprises at least one soluble UV-A-active and/or UV-B-active organic UV-screening agent.

12. Composition according to Claim 11, in which the soluble organic UV-screening agents are chosen especially from anthranilates; cinnamic derivatives; dibenzoylmethane derivatives; salicylic derivatives; camphor derivatives; triazine

derivatives; benzophenone derivatives other than those of formula (I); β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; screening polymers and screening silicones; dimers that are α-alkylstyrene derivatives; 4,4-diarylbutadienes; and mixtures thereof.

**13.** Composition according to Claim 12, in which the soluble organic UV-screening agents are chosen from:

- ethylhexyl salicylate,
- octocrylene,
- ethylhexyl methoxycinnamate,
- butylmethoxydibenzoylmethane,
- phenylbenzimidazolesulfonic acid,
- benzophenone-3,
- benzophenone-4,
- benzophenone-5,
- 4-methylbenzylidenecamphor,
- disodium phenyldibenzimidazoletetrasulfonate,
- anisotriazine,
- ethylhexyl triazone,
- diethylhexyl butamido triazone,
- 2,4,6-tris(diisobutyl 4'-aminobenzalmalonate)-s-triazine,
- drometrizole trisiloxane,
- 1,1-dicarboxy(2,2'-dimethylpropyl)-4,4-diphenyl-butadiene,

and mixtures thereof.

**14.** Composition according to any one of Claims 1 to 13, **characterized in that** it also comprises coated or uncoated metal oxide pigments or nanopigments.

**15.** Composition according to Claim 14, **characterized in that** the said pigments or nanopigments are chosen from coated or uncoated titanium oxide, zinc oxide, iron oxide, zirconium oxide and cerium oxide, and mixtures thereof.

**16.** Composition according to any one of Claims 1 to 15, **characterized in that** it also comprises at least one cosmetic adjuvant chosen from fatty substances, organic solvents, ionic or nonionic thickeners, softeners, antioxidants, free-radical scavengers, opacifiers, stabilizers, emollients, silicones, α-hydroxy acids, antifoams, moisturizers, vitamins, insect repellents, fragrances, preserving agents, surfactants, antiinflammatory agents, substance P antagonists, fillers, polymers, propellants, acidifying or basifying agents, and dyes.

**17.** Composition according to any one of Claims 1 to 16, **characterized in that** it is a composition for protecting the human epidermis or an antisun composition and **in that** it is in the form of a nonionic vesicular dispersion, an emulsion, in particular an emulsion of oil-in-water type, a cream, a milk, a gel, a cream gel, a suspension, a dispersion, a powder, a solid stick, a mousse or a spray.

**18.** Composition according to any one of Claims 1 to 17, **characterized in that** it is a composition for making up the eyelashes, the eyebrows or the skin and **in that** it is in solid or pasty, anhydrous or aqueous form or in the form of an emulsion, a suspension or a dispersion.

**19.** Composition according to any one of Claims 1 to 17, **characterized in that** it is a composition for protecting the hair against ultraviolet rays and **in that** it is in the form of a shampoo, a lotion, a gel, an emulsion or a nonionic vesicular dispersion.

**20.** Use of a composition as defined in Claims 1 to 17, for the manufacture of cosmetic or dermatological compositions for protecting the skin and/or the hair against ultraviolet radiation, in particular solar radiation.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1046391 A
- DE 10012408
- WO 03039507 A
- GB 2303549 A
- EP 893119 A
- WO 9522959 A
- US 5687521 A
- US 5373037 A
- US 5362881 A
- US 5237071 A
- US 5166355 A
- DE 19726184
- US 4367390 A
- EP 863145 A
- EP 517104 A
- EP 570838 A
- EP 796851 A
- EP 775698 A
- EP 878469 A
- EP 933376 A
- EP 669323 A
- US 2463264 A
- WO 9304665 A
- DE 19855649
- EP 0967200 A
- DE 19746654
- DE 19755649
- EP 1008586 A
- EP 0518772 A
- EP 0518773 A
- FR 2315991
- FR 2416008

### Littérature non-brevet citée dans la description

- **BANGHAM ; STANDISH ; WATKINS.** *J. Mol. Biol.,* 1965, vol. 13, 238